# EUROPEAN PATENT APPLICATION

(11) **EP 2 224 002 A1**
(43) Date of publication of application: **01.09.2010**
(21) Application number: 08855932.3
(22) Date of filing: 05.12.2008
(51) Int. Cl.: C12N 15/11, A61K 38/00, A61P 25/28

(54) **NP1 ACTIVITY REGULATING ELEMENTS WHICH CAN BE USED IN THE PRODUCTION OF DRUGS FOR THE TREATMENT OR PREVENTION OF HUMAN NEURODEGENERATIVE DISEASES, RESULTING DRUGS AND USE THEREOF**

(30) Priority: 05.12.2007 ES 200703249
(71) Applicant: Consejo Superior De Investigaciones Científicas, 28006 Madrid (ES)
(72) Inventor: TRULLAS OLIVA, Ramón, E-08036 Barcelona (ES); ENGUITA MARTINEZ, Marta, E-08036 Barcelona (ES); ABAD FERNANDEZ, Mº Alba, E-08036 Barcelona (ES)
(74) Representative: Pons Ariño, Angel
(86) International application number: PCT/ES2008/070229
(87) International publication number: WO 2009/071732

(57) **Abstract**

The invention relates to a series of compounds that can be used for the production of drugs or pharmaceutical compositions for the treatment or prevention of human neurological diseases, preferably neurodegenerative diseases, which inhibit the activity of the human NP1 protein which acts as an inducer of neurodegeneration and neural apoptosis. Said compounds include RNAi inhibitors of the gene expression of the NP1 gene and specific antibodies of the human NP1 protein, as well as peptides derived from same, which can be used in passive and active immunisation methods respectively against said diseases.

## Description

### SECTOR OF THE ART

Methods for prevention and treatment of neurodegenerative diseases, for example Alzheimer's and convulsive disorders.

### STATE OF THE ART

Prior evidence suggests that the biochemical mechanisms that cause the degeneration and pathological death of differentiated and mature neurones in various neurodegenerative disorders are the same as those comprising the intrinsic cell suicide programme or apoptosis one of the functions of which is to induce "de novo" synthesis of lethal proteins to eliminate surplus cells during brain development ¹⁻³.

In mature neurons it has been well established that the reduced neuronal activity sets in motion the intrinsic cell death programme that provokes "de novo" synthesis of lethal proteins that cause apoptotic neurodegeneration ⁴. Identifying these lethal pro-apoptotic proteins that are synthesised from new before neurons reach an irreversible point in the process of dying has been the strategy that has guided the research of our laboratory so as to obtain new targets for the development of neuroprotective treatments.

With this objective, gene expression has been investigated during the initial phase of the neuronal death programme produced by the reduction in synaptic activity. By using a differential gene expression analysis technique ⁵, it has been proven that Neuronal Pentraxin 1 (NP1) is a lethal pro-apoptotic protein that is synthesised in greater amounts when neuronal activity declines. Prior studies indicate that NP1 forms part of the intrinsic death programme and contributes to the apoptotic neurodegeneration that gives rise to reduced neuronal activity ⁶. Originally, NP1 was identified and isolated as a protein that binds in a calcium-dependent manner with taipoxin, a toxin of snake venom ⁷. The NP1 gene encodes a glycoprotein with an apparent molecular mass of approximately 50 kDa, whose sequence of amino acids predicts that it is secreted. NP1 expression is restricted to the nervous system ⁷.

NP1 forms part of the family of Pentraxin proteins, which receives its name due to the capacity to form pentameters. This family is composed of 8 proteins that can be subdivided into two structural classes according to their size; short pentraxins (approx. 200 amino acids) and long pentraxins (approx. 400 amino acids) ⁸. Short pentraxins, identified in the first place a long time ago, form part of the acute-phase response of the innate immune system and include C-reactive protein (CRP) and the serum amyloid P component (SAP). Long pentraxins, which have been identified more recently, share with the short ones the Pentraxin domain on the C-terminal side, but on their Amino-terminal side are very different from each other. There are at least three long Pentraxins that are expressed in the nervous system: Neuronal Pentraxin 1 (NP1), the pentraxin related to neuronal activity or Neuronal Pentraxin 2 (Narp or NP2) and the neuronal pentraxin receptor (NPR) ^{7;9-11}. From the study of the sequences it is predicted that NP1 and NP2 are secretion proteins, whereas NPR is identified as a type II transmembrane protein without an intracellular domain ^{9;12}. The physiological function of neuronal pentraxins has not yet been established. However, based on the homology of NP1 to shorter pentraxins such as C-reactive protein and serum amyloid P protein, it was initially suggested that the function of NP1 is to capture waste material during synaptic remodelling (Schlimgen et al., 1995; Omeis et al., 1996; Kirkpatrick et al., 2000). At the same time, more recent studies by Paul Worley's group (J. Hopkins) have demonstrated that Narp/NP2 has synaptogenic effects and participates in synaptic remodelling phenomena ^{13;14}.

Studies by the group of Paul Worley and Richard Huganir have shown that NP1 and NP2 regulate the grouping of the AMPA subtype of glutamate receptors in excitor synapses, binding to them in an extracellular domain. The amino terminal half of NP1 encodes various coiled-coil domains which are essential for NP1 multimerisation with itself or with other proteins¹³. Also, the carboxyl terminal half of NP1 encodes a calcium-dependent lectin domain that recognises oligosaccharides in glycoproteins or glycolipids. To date it has been proven that NP1 binds to the AMPA subtypes of glutamate receptors ¹⁴, but other proteins that interact with NP1 have not yet been identified, with the exception of NP2 and the AMPA receptors. Up until very recently, neither the function nor any interaction of the pentraxin receptor NPR was known either. However a very recent study has shown that NPR has a CHROMO (chromatin-organisation modifier domain) which is a domain of interaction with other proteins and DNA. This study demonstrates that NPR through its CHROMO domain binds to the intracellular domain of the tyrosine phosphatase receptor PTPRO which is involved in axonal guidance and growth ¹⁵. This result allows us to consider that the possible effects on the neuritogenesis of NP1 may be mediated by interaction with NPR and the tyrosine phosphatase receptor PTPRO associated thereto.

At the same time, while investigating the influence of the various intracellular signalling pathways that control cell survival and death on the expression of NP1 it has been proven that overexpression of NP1 during the apoptotic death process is induced through an activating phosphorylation of GSK3 kinase activity. Neither the signalling pathways involved in cell death, such as stress-activated protein kinases (SAP kinases), c-jun N-terminal (JNK) or p38, nor the trophic signalling pathway PI3K/AKT activated by IGF, regulate the expression of NP1 ¹⁶. The finding that NP1 expression is regulated by the GSK3 kinase provides evidence indicating that neuritic degeneration induced by NP1 shares a common mechanism with amyloid beta-induced neurodegeneration. Various studies have shown that amyloid beta provokes an increase in the activity of GSK3 kinase ¹⁷ and that GSK3 is increased in a brain with Alzheimer's disease (see review in ¹⁸). On a separate note, there is well-founded evidence that the increase in GSK3 kinase activity produces neuritic retraction, whereas reduced GSK3 activity produces synaptogenesis ¹⁹⁻²².

Most procedures and techniques of therapeutic intervention that to date have been directed at preventing the neuronal damage in neurological disorders aim to diminish the toxic stimuli that cause the disorder ²³. For example, in Alzheimer's disease, there is a large amount of results which indicate that soluble oligomers of the amyloid beta protein (Ab) are the cause of loss of memory, dementia and the neurodegeneration typical of the disease ²⁴⁻²⁶. Based on this evidence, most strategies to treat and prevent Alzheimer's disease aim to reduce the amount of Ab protein, whether through pharmacological modulation of the secretases of Ab or through active or passive immunotherapy to reduce the amount of soluble oligomers of Ab ²³.

It is a while now since it was postulated that neurotoxicity induced by the Ab peptide is responsible for synaptic alterations and the neuronal degeneration observed in Alzheimer's disease ²⁷. Despite the fact that the apoptotic hypothesis of neuronal degeneration in an Alzheimer brain is still under debate ²⁶, various histological analyses of brains from patients suffering from the disease have identified neurons with apoptotic morphology ²⁸. Moreover, various studies have shown that the Ab peptide induces the apoptotic death programme in primary neuron cultures ^{29;30}.

Previous experiments have proven that Neuronal Pentraxin 1 (NP1) forms part of the apoptotic neuronal death programme ^{6;16}. Thus, the results show that the reduction in neuronal activity caused by potassium deprivation produces a large increase in the expression of NP1 before causing neuronal death. These results allowed a new function to be proposed for NP1: that NP1 is part of the gene programme of apoptotic death that is induced by reduction of the activity ⁶.

As distinct from the protein NP1, it has been proven that the expression of another pentraxin of the family of long pentraxins, Narp/NP2, is not altered by reduction in activity or other neurotoxic stimuli. On the contrary, the expression of Narp/NP2 only increases when neuronal activity increases in situations such as long-term potentiation or convulsions ¹¹. Based on these observations, the hypothesis has been put forward that neuronal pentraxins NP1/NP2 form part of a gene switch operated by neuronal activity. On a separate note, there is evidence indicating that Ab induces neuritic degeneration in primary cultures of hippocampus neurons through a mechanism in common with the apoptotic process ³¹.

At the same time, recently it has been proven that Ab production depends on neuronal activity and that in turn, the Ab protein reduces excitatory synaptic neurotransmission ^{33;34}

### DESCRIPTION OF THE INVENTION

### Brief Description

One aspect of the invention consists of a compound which can be used in the production of drugs or pharmaceutical compositions for the treatment of human neurological diseases, preferably neurodegenerative diseases, hereinafter compound of the invention, comprising an inhibitor compound or agent of the activity of human NP1 protein.

Therefore, one particular aspect of the invention consists of a compound of the invention wherein the inhibitor compound is a nucleic acid or polynucleotide that prevents or diminishes the expression of the gene encoding human NP1 protein and that includes, at least, a sequence of nucleotides selected between:
a) an antisense sequence of nucleotides specific to the sequence of the gene or mRNA of the NP1 protein,
b) a specific ribozyme of the mRNA of the NP1 protein,
c) a specific aptamer of the mRNA of the NP1 protein,
d) an interference RNA (shRNAi) specific to the mRNA of the NP1 protein, and
e) a microRNA specific to the mRNA of the NP1 protein.

Another particular aspect of the invention consists of a compound of the invention wherein the inhibitor compound is a vector, hereinafter the expression vector, which comprises a nucleic acid or polynucleotide of the invention that prevents or diminishes expression of the gene encoding the human NP1 protein, whether an expression vector or a transfer vector.

Another particular aspect of the invention consists of a compound of the invention wherein the inhibitor compound is an antibody specific to the human NP1 protein and functionally active that prevents or diminishes the neurodegenerative activity of the human NP1 protein, whether monoclonal or polyclonal.

Another particular embodiment of the invention consists of the antibody of the invention wherein it is a preferably polyclonal antibody, specific to an epitope composed of the sequence of amino acids corresponding to the X-Y sequence of the human NP1 protein (SEQ ID N018).

Another particular aspect of the invention consists of a peptide or epitope of the human NP1 protein, hereinafter peptide of the invention, composed of a fragment of the zone comprised between amino acids 24 to 229 of the NP1 protein, preferably of a size of 15 amino acids.

Another aspect of the invention consists of the use of an inhibitor compound or agent of the activity of the human NP1 protein, hereinafter use of the invention, in the production of a drug or pharmaceutical composition for the treatment of human neurological diseases, preferably neurodegenerative diseases, more preferably Alzheimer's disease.

Another aspect of the invention consists of a pharmaceutical composition or drug for the treatment of neurological diseases, preferably neurodegenerative, hereinafter pharmaceutical composition of the invention, which comprises a therapeutically effective amount of an inhibitor compound or agent of the human NP1 protein, together with, optionally, one or more adjuvants and/or pharmaceutically acceptable vehicles.

Another particular aspect of the invention consists of the pharmaceutical composition of the invention wherein the inhibitor compound is a nucleic acid or polynucleotide that prevents or diminishes the expression of the gene encoding the human NP1 protein and that comprises, at least, one sequence of nucleotides selected between:
a) an antisense sequence of nucleotides specific to the sequence of the gene or mRNA of the NP1 protein,
b) a ribozyme specific to the mRNA of the NP1 protein,
c) an aptamer specific to the mRNA of the NP1 protein,
d) an interference RNA (iRNA) specific to the mRNA of the NP1 protein, and
e) an interference microRNA (iRNA) specific to the mRNA of the NP1 protein.

Another particular aspect of the invention consists of the pharmaceutical composition of the invention wherein the inhibitor compound is an antibody specific to the NP1 protein. This therapeutic composition can be used in a therapeutic procedure of passive immunisation of patients with a neurodegenerative disease, preferably with Alzheimer's disease.

Another particular aspect of the invention consists of the pharmaceutical composition of the invention wherein the inhibitor compound is a peptide of the invention and can be used in a therapeutic procedure of active immunisation of patients with a neurodegenerative disease, preferably with Alzheimer's disease.

Another object of the invention comprises the use of the pharmaceutical composition of the invention in a method for treating a mammal, preferably a human being, affected by a neurological disease, preferably a neurodegenerative disease, more preferably Alzheimer's disease, hereinafter use of the pharmaceutical composition of the invention, consisting of the administration of said therapeutic composition that inhibits the neuropathological process.

### Detailed Description

The invention is based on the fact that the inventors have observed that the reduction in the expression of one of the proteins of the intrinsic apoptotic cell death programme, specifically neuronal pentraxin 1, the NP1 protein, induces the increase in synaptic proteins and an increase in neuronal excitability making it possible to reduce or stop the processes of neurodegeneration that occur in mammals, preferably humans, and more preferably in Alzheimer's disease. Thus, when neuronal activity decreases, as happens when a lot of Ab accumulates, the amount of NP1 increases and the process of synaptic reduction and apoptotic neurodegeneration begins.

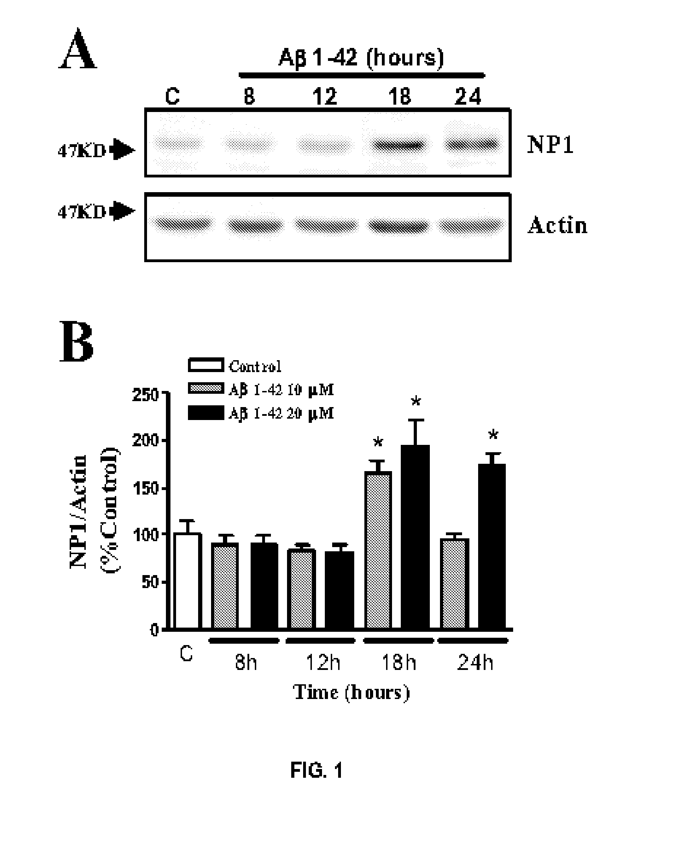

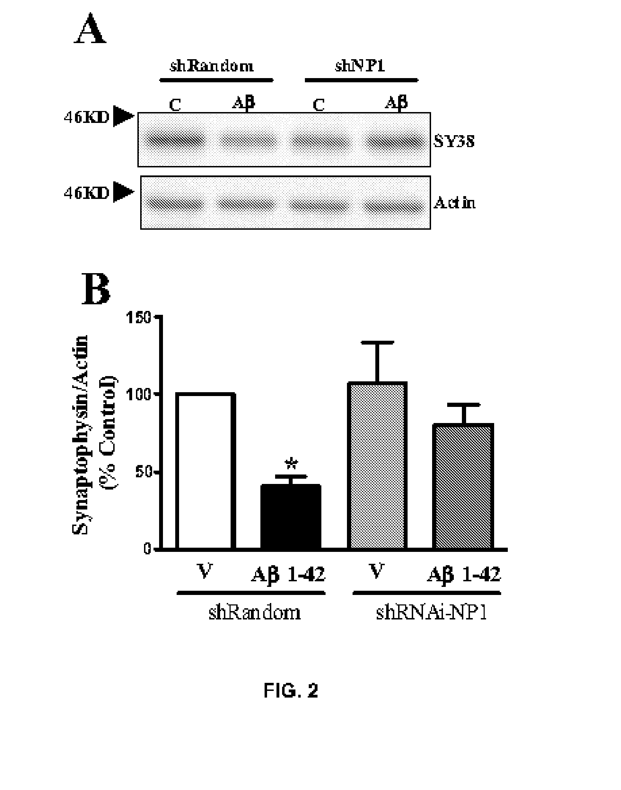

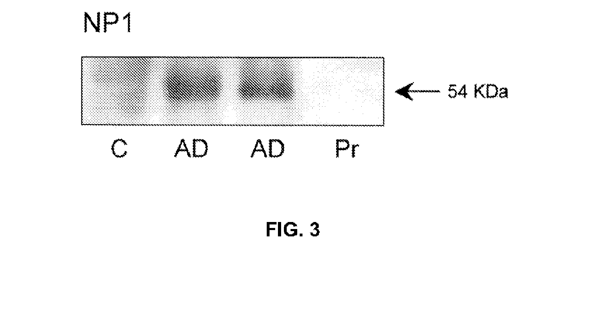

More specifically, the results obtained demonstrate that exposure of primary cultures of cortical neurons to the Ab peptide increases the expression of NP1 before inducing apoptotic death (Figure 1). In addition, it has also been proven that NP1 is an essential factor for producing neurotoxicity by Ab, and that in turn provokes loss of synapsis and neuronal damage. The loss of synaptic contacts between neurons is directly related to loss of memory. When the increase in NP1 is blocked through gene silencing by interference RNA damage to synapsis is impeded and the appearance of the neurotoxic effects of Ab is prevented (Figure 2). However, in the absence of Ab, if the neurons are treated with NP1 the neuropathological process induced by Ab is reproduced. Additionally, it has also been observed that the amount of NP1 is much higher in the brains of patients with Alzheimer's than in control brains (Figure 3). In brains diagnosed with Alzheimer's NP1 is located in the damaged neuronal dendrites, where it associates with synaptic proteins such as SNAP-25 and synaptophysin. These results establish that NP1 plays a fundamental role in the neurotoxicity provoked by the soluble oligomers of the amyloid beta protein in Alzheimer's disease.

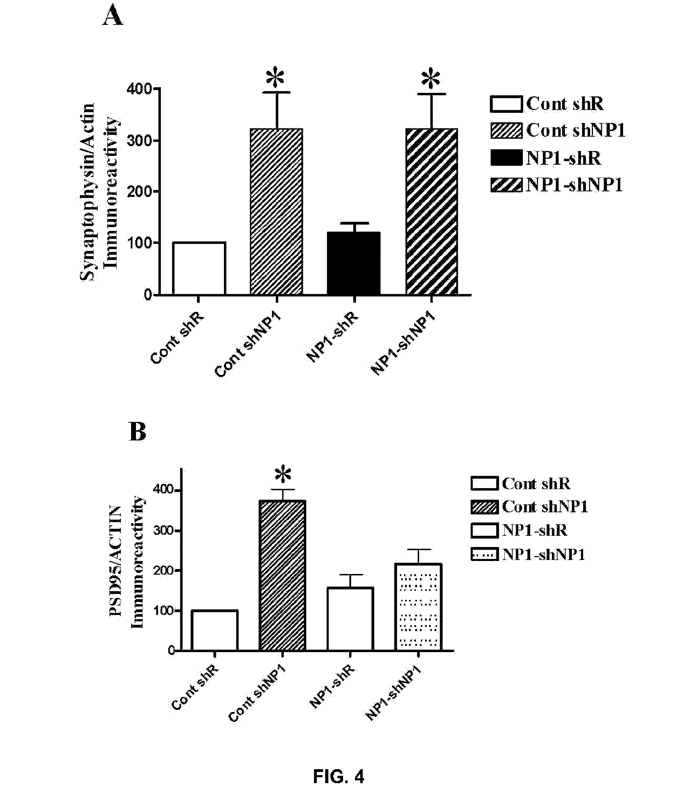

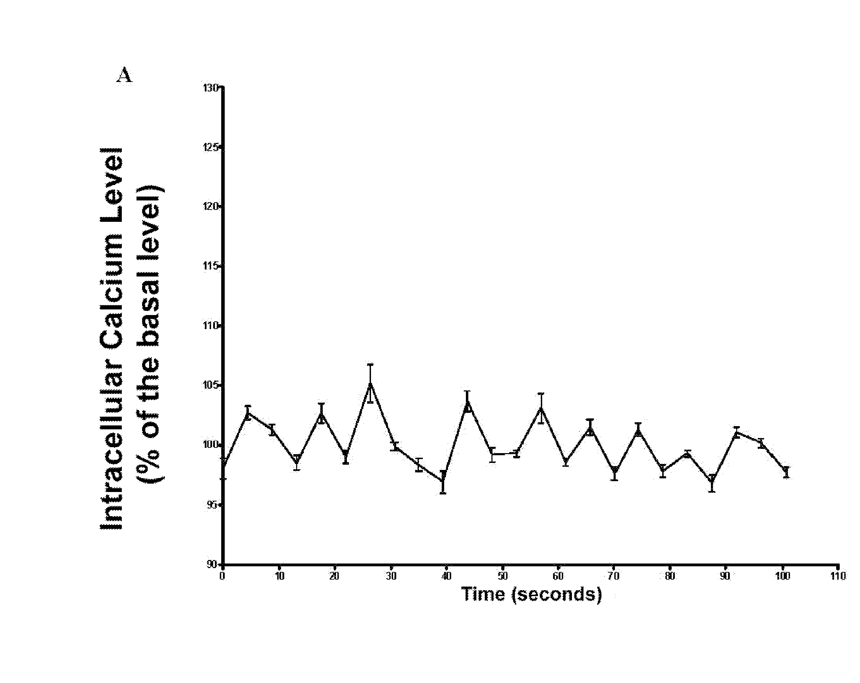

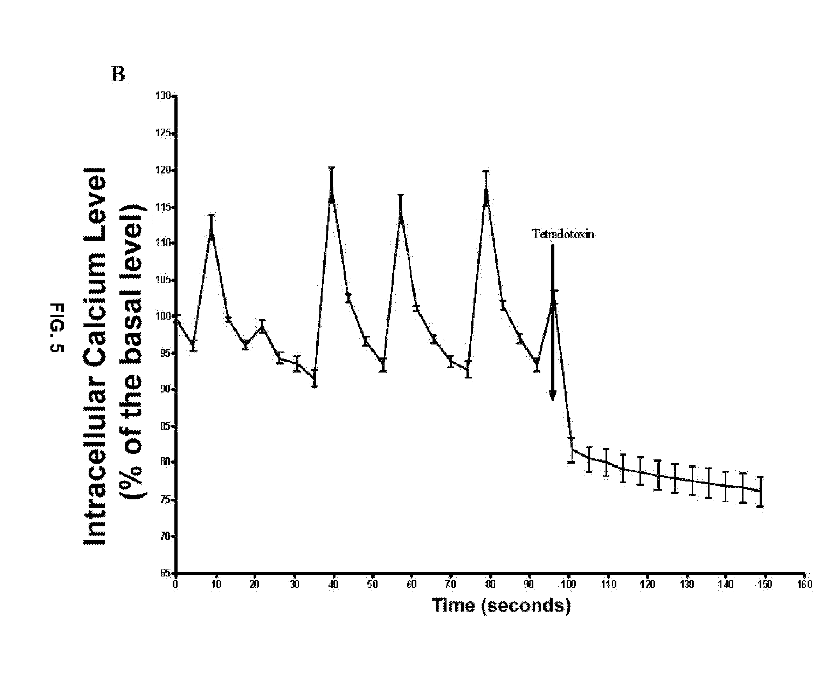

Similarly, the decrease in the expression of the NP1 protein through gene silencing by RNAi produces a significant increase in the synaptic proteins PSD95 and synaptophysin and therefore an increase in the number of excitor synapses, which constitutes evidence of the recovery or maintenance of memory-related parameters (Figure 4); and at the same time it produces an increase of neuronal excitability in primary cultures of cortical neurons (Figure 5).

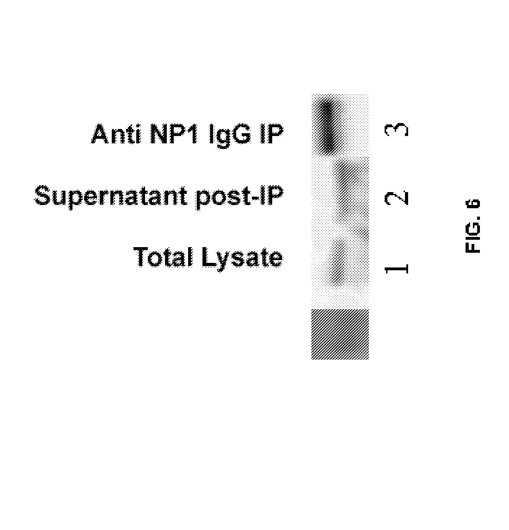

It has been verified that antibodies directed against epitopes of the human NP1 protein (NPTX1_HUMAN, SwissProt primary accession number Q15818) recognise the native and denatured forms of NP1 and are capable of immunoprecipitating NP1 (Figure 6), enabling postulation of their use in therapeutic processes of passive immunisation of patients with a neurodegenerative disease, preferably with Alzheimer's disease. Also, peptides derived from the sequence of human NP1 protein, more preferably, peptides whose sequence is found within the zone between amino acids 24 to 229 of NP1, can be used as drugs to induce active immunisation (generation of endogenous antibodies) in patients with a neurodegenerative disease, preferably with Alzheimer's disease.

As distinct from Alzheimer's disease, epilepsy is a chronic neurological disorder characterised by the recurrent appearance of spontaneous convulsions. These convulsions are associated to the neuronal hyperexcitability of specific areas of the brain produced by excessive or asynchronous neuronal activity. Mention as an additional comment justification of the potential applications of these same elements in the treatment of epilepsy.

In summary, the results of the invention allow new therapeutic approaches to neurodegenerative diseases to be developed, through use of pharmaceutical compositions comprising active principles that inhibit the expression of the human NP1 protein. At the same time, regulation of NP1 expression can represent an effective therapeutic treatment for other chronic neurological disorders such as convulsive disorders.

Therefore, one aspect of the invention consists of a compound which can be used in the production of drugs or pharmaceutical compositions for the treatment of human neurological diseases, preferably neurodegenerative diseases, hereinafter compound of the invention, comprising an inhibitor compound or agent of the activity of the human NP1 protein.

As used in the invention, the term "inhibitor or antagonist compound/agent" refers to a molecule that when it binds or interacts with human NP1 protein, or with functional fragments thereof, diminishes or eliminates the intensity or duration of the neurodegenerative biological activity of said protein. This definition includes also those compounds which prevent or diminish the expression of the gene encoding the human NP1 protein, in other words, which prevent or diminish the transcription of the gene, the maturation of mRNA, the translation of mRNA and post-translation modification. An inhibitor agent can consist of a peptide, a protein, a nucleic acid or polynucleotide, a carbohydrate, an antibody, a chemical compound or any other type of molecule that diminishes or eliminates the effect and/or function of the human NP1 protein.

By way of illustration, said polynucleotide can be a polynucleotide encoding an antisense sequence of nucleotides specific to the sequence of the gene or mRNA of the NP1 protein, or a polynucleotide encoding a ribozyme specific to the mRNA of the NP1 protein, or a polynucleotide encoding an aptamer specific to the mRNA of the NP1 protein, or a polynucleotide encoding an interference RNA ("small interference RNA" or siRNA) specific to the mRNA of the NP1 protein, or a polynucleotide encoding a microRNA specific to the mRNA of the human NP1 protein.

As used in the invention, "human NP1 protein" refers to a protein with the following reference sequence: NPTX1_HUMAN, SwissProt primary accession number Q15818, or a functionally equivalent variant thereof.

"Functionally equivalent variant" or "variant" is understood as meaning, in the context of the invention, any protein that can be obtained from the abovementioned human NP1 protein through substitution, deletion or insertion or one or more amino acids and that substantially maintains the function of the original protein. Determination of the function of human NP1 can be carried out using conventional methods well-known to an expert in the art, among which are those used in the invention. This variant comprises fragments of the human NP1 protein with neurodegenerative activity.

In the case of variants through substitution, the substitutions are preferably conservative substitutions, in other words, the amino acids are substituted by others of similar characteristics in respect of the properties of their side chain. Thus, conservative substitutions include substitutions within the groups of amino acids according to table 1.

| Type of side chain | Amino acid |
|---|---|
| Aliphatic apolar or slightly polar | Ala, Ser, Thr, Pro, Gly |
| Polar with neutral positive charge | His, Arg, Lys |
| Polar with neutral negative charge and the corresponding amides | Asp, Asn, Glu, Gln |
| Aromatic | Phe, Tyr, Trp |
| Aliphatic large and apolar | Met, Leu, He, Val, Cys |

Additionally, one or more of the amino acids of the variants of the invention may be substituted by non-conventional natural or synthetic amino acids such as for example, beta-amino acids, 2-aminoadipic acid, alpha-asparagine, 2-aminobutanoico acid, 2-aminocaproic acid, alpha-glutamine, alpha-methylalanine, 2-aminopimelic acid, gamma-amino-beta-hydroxybenzenepentanoic acid, 2-aminosuberic acid, 2-carboxyazetidine, beta-alanine, beta-aspartic acid, 3,6 diaminohexanoic acid, butyric acid, 4-amino 4-amino-3-hydroxybutyric acid, gamma-amino-beta-hydroxycyclohexanepentanoic acid, N5-aminocarbonylornithine, 3-sulfoalanine, 2,4 diaminobutyric acid, diaminopimelic acid, 2,3 diaminopropanoic acid, 2,7 diaminosuberic acid, S-ethylthiocysteine, gamma-glutamic acid, gamma-carboxyglutamic acid, pyroglutamic acid, homoarginine, homocysteine, homohistinba, homoserine, 2-hydroxyisovaleric acid, 2-hydroxypentanoic acid, 5-hydroxylysine, 4-hydroxyproline, 2-carboxyoctahydroindole, 3-carboxyisoquinoline, isovaline, 2-hydroxypropanoic acid, mercaptoacetic acid, mercaptobutyric acid, 4-methyl-3-hydroxyproline, mercaptopropanoic acid, norleucine, nortyrosine, norvaline, ornithine, penicillamine, 2-phenylglycine, 2-carboxypiperidine, sarcosine, 1-amino-1-carboxycyclopentane, statin, 3-thienylalanine, epsilon-N-trimethyl-lysine, 3- thiazolealanine, alpha-amino-2,4-dioxo pyrimidine propionic acid.

In addition, the invention contemplates variants of the peptides of the invention wherein one or more amino acids have sustained modifications in their side chain. Examples of side chain modifications contemplated in the invention include modifications of amino groups such as alkylation, amidination, acylation, carbamylation, trinitrobenzylation, pyridoxylation, modifications of the guanidine group of arginine residues consisting of the formation of heterocyclic condensates; modification of the carboxyl groups through amidation, modification of tyrosines through methoxylation, modification of the imidazole ring of histidine through alkylation or N-carboxyethylation, modifications of proline through hydroxylation in position 4.

Alternatively, the invention contemplates variants of the peptides of the invention through glycosylation, in other words, the addition of glycan groups either on the serine and/or threonine side chain (O-glycosylation) or on the asparagine and/or glutamine side chain (N-glycosylation). The glycans that can be incorporated into the polypeptides of the invention include a variable number of glucidic units (mono-, di-, tri, tetrasaccharides and successively). The monosaccharides that form in glycan include D-allose, D-altrose, D-glucose, D-mannose, D-gulose, D-idose, D-galactose, D-talose, D-galactosamine, D-glucosamine, D-N-acetylgucosamine, D-N-acetylgalactosamine, D-fucose or D-arabinose.

Alternatively, the invention contemplates variants of the polypeptides of the invention that include the D stereoisomers of at least one of the amino acids comprising the peptide chain so as to thus give rise to the retro-inverse isomers.

In another mode of embodiment, the invention contemplates peptidomimetics of the polypeptides of the invention, in other words, variants wherein one or more of the peptide bonds have been replaced by an alternative type of covalent bond. Said peptidomimetics are **characterised in that** they demonstrate greater stability through being more resistant to proteases. Modifications of the peptide skeleton include the substitution or insertion in the elements of the peptide bond (-NH-, -CH-, -CO-) of groups such as -O-, -S-, -CH2 instead of -NH-, -N-, -C-alkyl p -BH- instead of -CHR and -CS-, -CH2-, -SOn-, -P=O(OH)- or -B(OH)- instead of -CO-. In addition, it is possible to increase the stability of the peptides of the invention using groups that block the N-terminal end such as t-butyloxycarbonyl, acetyl, succinyl, methoxysuccinyl, suberyl, adipyl, dansyl, benzyloxycarbonyl, fluorenylmethoxycarbonyl, methoxyadipyl, methoxyadipyl, methoxysuberyl and 2,3-dinitrophenyl. Alternatively or simultaneously, it is possible to modify the C-terminal end of the peptides through amidation.

Determination of the degree of identity between the variants and the polypeptides defined in sequences 1 to 19 is carried out using software algorithms and methods well-known by an expert in the art. Preferably, the identity between two sequences of amino acids is determined using the BLASTP algorithm (BLAST Manual, Altschul, S., et al, NCBI NLM NIH Bethesda, Md. 20894, Altschul, S., et al., J. Mol. Biol. 21 5: 403-410 (1990). Preferably, the polypeptides of the invention show an identity of the sequence with the polypeptides defined in the sequences of SEQ ID NO:1 to 19 of at least 50%, at least 60%, at least 70%, at least 80%, at least 90%, at least 91%, at least 92%, at least 93%, at least 94%, at least 95%, at least 96%, at least 97%, at least 98% or at least 99%.

Therefore, a particular aspect of the invention consists of a compound of the invention wherein the inhibitor compound is a nucleic acid or polynucleotide that prevents or diminishes the expression of the gene encoding human NP1 protein and that includes, at least, a sequence of nucleotides selected between:
a) an antisense sequence of nucleotides specific to the sequence of the gene or mRNA of the NP1 protein,
b) a ribozyme specific to the mRNA of the NP1 protein,
c) an aptamer specific to the mRNA of the NP1 protein,
d) an interference RNA (shRNAi) specific to the mRNA of the NP1 protein, and
e) a microRNA specific to the mRNA of the NP1 protein.

On a separate note, these techniques of gene inhibition, and more specifically, transfer of the compounds - antisense oligonucleotides, iRNA, ribozymes or aptamers - can be carried out through the use of nanoparticles that increase the success of said transfer (Lu PV and Woodle MC, Adv Genet 54: 117-42, 2005; Hawker CJ and Wooley KL, Science 19 (309): 1200-5, 2005).

A more particular aspect of the invention consists of an RNAi that binds preferably to the fragment sequence of RNAm of NP1 GAGCTCCAGATCATGAGAA (SEQ ID N01; this sequence corresponds to the bases 1004 to 1022 of the mRNA of rat NP1 (Genbank Accession number U18772)) or to another fragment that comprises this sequence.

Thus, a particular embodiment consists of an shRNAi against NP1 (SEQ ID N01) expressed by the following pair of nucleotides: 5'-gatcccc GTACAGCCGCCTCAATTCT ttcaagaga AGAATTGAGGCGGCTGTAC ttttt-'3 (SEQ ID N04, sense) and 5'-agctaaaaa GTACAGCCGCCTCAATTCT tctcttgaa AGAATTGAGGCGGCTGTAC ggg-3' (SEQ ID N05, antisense).

Another particular embodiment consists of an shRNAi against the homologous form of the SEQ ID N01 corresponding to the bases 602 to 620 of the mRNA of human NP1 (Genbank Accession number NM_002522) and expressed by the following pair of nucleotides: 5'-gatcccc GCAGTACAGCCGCCTCAAT ttcaagaga ATTGAGGCGGCTGTACTGC ttttt-'3 (SEQ ID N06, sense), and 5'-agctaaaaa GCAGTACAGCCGCCTCAAT tctcttgaa ATTGAGGCGGCTGTACTGC ggg-3' (SEQ ID N07, antisense).

Another more particular aspect of the invention consists of an RNAi that binds preferably to the fragment sequence of RNAm of NP1 GCGGACCAACTACATGTAT (SEQ ID N02; this sequence corresponds to the bases 1259 to 1277 of the mRNA of rat NP1 (Genbank Accession number U18772)) or to another fragment that comprises this sequence.

Another particular embodiment consists of an shRNAi against NP1 (SEQ ID N02) expressed by the following pair of nucleotides: 5'-gatcccc GCGGACCAACTACATGTAT ttcaagaga ATACATGTAGTTGGTCCGC ttttt-'3 (SEQ ID N08, sense) and 5'-agctaaaaa GCGGACCAACTACATGTAT tctcttgaa ATACATGTAGTTGGTCCGC ggg-3' (SEQ ID N09, antisense).

Another particular embodiment consists of an shRNAi against the homologous form of SEQ ID N01 corresponding to the bases 860 to 878 of the mRNA of human NP1 (Genbank Accession number NM_002522) and expressed by the following pair of nucleotides: 5'-gatcccc GCGGACCAACTATATGTAT ttcaagaga ATACATATAGTTGGTCCGC ttttt-'3 (SEQ ID NO10, sense), and 5'-agctaaaaa GCGGACCAACTATATGTAT tctcttgaa ATACATATAGTTGGTCCGC ggg-3' (SEQ ID N011, antisense).

Another more particular aspect of the invention consists of an RNAi that binds preferably to the fragment sequence of RNAm of NP1 GAGATACTCATTAACGACA (SEQ ID N03; this sequence corresponds to the bases 1434 to 1452 of the mRNA of rat NP1 (Genbank Accession number U18772)) or to another fragment that comprises the latter.

Another particular embodiment of the invention consists of an shRNAi against NP1 (SEQ ID N03) expressed by the following pair of nucleotides: 5'-gatcccc GAGATACTCATTAACGACA ttcaagaga TGTCGTTAATGAGTATCTC ttttt-3' (SEQ ID NO12, sense) and 5'-agctaaaaa GAGATACTCATTAACGACA tctcttgaa TGTCGTTAATGAGTATCTC ggg-3' (SEQ ID N013, antisense).

Another particular embodiment of the invention consists of an shRNAi against the homologous form of SEQ ID N01 corresponding to the bases 1035 to 1053 of the mRNA of human NP1 (Genbank Accession number NM_002522) and expressed by the following pair of nucleotides: 5'-gatcccc GAGATCCTCATCAATGACA ttcaagaga TGTCATTGATGAGGATCTC ttttt- 3' (SEQ ID NO14, sense), and 5'-agctaaaaa GAGATCCTCATCAATGACA tctcttgaa TGTCATTGATGAGGATCTC ggg-3' (SEQ ID N015, antisense).

The nucleotide sequences a)-e) mentioned above prevent the expression of the gene in mRNA or of the mRNA in the NP1 protein, and, therefore, annul its biological function, and can be developed by an expert in the field of genetic engineering based on existing knowledge in the state of the art in relation to transgenesis and the annulment of gene expression (Clarke, A.R. (2002) Transgenesis Techniques. Principles and Protocols, 2a Ed. Humana Press, Cardiff University; Patent US20020128220. Gleave, Martin. TRPM-2 antisense therapy; Puerta- Ferández E et al. (2003) Ribozymes: recent advances in the development of RNA tools. FEMS Microbiology Reviews 27: 75-97; Kikuchi, et al., 2003. RNA aptamers targeted to domain II of Hepatitis C virus IRES that bind to its apical loop region. J. Biochem. 133, 263-270; Reynolds A. et al., 2004. Rational siRNA design for RNA interference. Nature Biotechnology 22 (3): 326-330).

The aforesaid polynucleotides can be used in a gene therapy process wherein through any technique or procedure integration thereof is allowed in the cells, preferably, the cells of a human diseased patient. This objective can be achieved through administration to the neuronal cells of a gene construct that comprises one of the aforesaid polynucleotides with a view to transforming said cells allowing its expression therein in such a way that expression of the NP1 protein is inhibited. Advantageously, said gene construct can be included within a vector.

As used in the invention, the term "vector" refers to systems used in the process of transferring an exogenous gene or a gene construct to the inside of a cell, thereby allowing the transfer of exogenous genes and gene constructs, such as, for example, an expression vector or a transfer vector. Said vectors can be non-viral or viral vectors (Pfeifer A, Verma IM (2001) Gene therapy: promises and problems. Annu Rev Genomics Hum Genet 2: 177-211) and their administration can be prepared by an expert in the art according to the needs and specificities of each case.

In general, the expression vector of the invention comprises, at least, the sequence of nucleotides of the invention, at least, a promoter that directs its transcription (*pT7, plac, ptrc, ptac, pBAD, ptet,* etc), to which it is operatively bound, and other necessary or appropriate sequences that control and regulate the transcription of the gene. Examples of appropriate expression vectors can be selected according to the conditions and needs of each specific case from among expression plasmids of microorganisms which can additionally contain markers that can be used to select the cells transfected or transformed with the gene or genes of interest. The choice of vector will depend on the host cell and the type of use required. Therefore, according to a particular mode of embodiment, said vector is a plasmid or a viral vector, for example, a lentivirus. Said vector can be obtained using conventional methods known by technicians in the field and in the same way different methods can be used for the transformation of microorganisms or eukaryote cells - chemical transformation, electroporation, microinjection, etc. - as described in various manuals [Sambrook, J., Fritsch, E. F., and Maniatis, T. (1989). Molecular cloning: a laboratory manual, 2nd ed. Cold Spring Harbor Laboratory, Cold Spring Harbor, N.Y.]. At the same time, a transfer vector can be comprised, by way of illustration without limiting the invention, in the following group: microspheres, liposomes, nanoparticles and dendrimers; which have the capacity to connect to the sequence of nucleotides of the invention, transferring it to the inside of the cells and subsequently releasing it.

Thus, another particular aspect of the invention consists of a compound of the invention wherein the inhibitor compound is a vector, hereinafter expression vector, which comprises a nucleic acid or polynucleotide of the invention that prevents or diminishes the expression of the gene encoding human NP1 protein, whether an expression vector or a transfer vector.

Another particular embodiment of the invention consists of a viral expression vector, preferably a lentivirus capable of expressing inside the cell a nucleotide that inhibits the expression of human NP1 protein, preferably the lentivirus vector pLVTHM-shRNAi-NP1 developed in the invention (see Material and Methods) which comprises, at least, one of the following pairs of sequences:
5 sequences SEQ ID NO 4 and 5,
6 sequences SEQ ID NO 6 and 7,
7 sequences SEQ ID NO 8 and 9,
8 sequences SEQ ID NO 10 and 11,
9 sequences SEQ ID NO 12 and 13, and
10 sequences SEQ ID NO 14 and 15.

Another particular aspect of the invention consists of a compound of the invention wherein the inhibitor compound is an antibody specific to human NP1 protein and functionally active that prevents or diminishes the neurodegenerative activity of the human NP1 protein, whether monoclonal or polyclonal.

As used in the invention, the term "functionally active antibody" refers to a recombinant antibody that maintains its capacity to bind to an antigen, including miniantibodies, which are defined as fragments derived from antibodies constructed using recombinant DNA technology, which, despite their smaller size, retain their capacity to bind to the antigen since they maintain at least one variable domain of immunoglobulin where the antigen-binding zones reside, and which belongs, by way of illustration without limiting the scope of the invention, to the following group: polyclonal anti-serums, purified molecules of IgG**,** supernatants or ascitic liquid containing monoclonal antibodies, fragments Fv, Fab, Fab' and F(ab')2, ScFvdiabodies, single domain recombinant antibodies (dAbs), humanised antibodies triabodies and tetrabodies. In the context of the invention, recombinant antibodies of single domain and/or immunoglobulin-type domains capable of independent binding and recognition are understood as meaning the heavy chain variable domains (VH), light chain variable domains (VL), the recombinant antibodies of camelids (VHH), the recombinant antibodies of humanised camelids, the recombinant antibodies of other camelised species, the single domain IgNAR antibodies of cartilaginous fish; in other words, include both domains that are naturally single domain (in the case of VHH and IgNAR), as well as antibodies which have been altered through engineering so that they are capable on their own of interacting with the antigen and improving their stability and solubility properties. This definition includes any modification of the recombinant antibodies such as their multimerisation or fusion to any molecule (e.g. toxins, enzymes, antigens, other antibody fragments, etc.).

The functionally active antibody may be obtained from a human being or an animal (e.g. camels, llamas, vicunas, mice, rats, rabbits, horses, nurse sharks, etc.) or through recombinant DNA techniques or chemical gene synthesis, and at the same time, includes both monoclonal as well as polyclonal antibodies.

Another more particular aspect of the invention consists of the antibody of the invention, whether monoclonal or polyclonal, specific to an epitope that is found between amino acids 24 to 229 of the sequence of the human NP1 protein (NPTX1_HUMAN, SwissProt primary accession number Q15818), preferably to epitopes or peptides of 15 amino acids in size.

Another particular embodiment of the invention consists of the antibody of the invention wherein it is a preferably polyclonal antibody, specific to an epitope made up of the sequence of amino acids corresponding to the sequence belonging, by way of illustration and without limiting the scope of the invention, to the following group: SEQ ID N018 and SEQ ID NO19. As commented in the examples, the area selected by the inventors (amino acids 24 and 229) is the most characteristic of NP1 and the one that distinguishes itself more from other proteins such as NP2 and NP3 and that predicts domains of interaction with other proteins, which makes this region a good therapeutic target, just like the homologous region to it in the human NP1 protein.

Another particular aspect of the invention consists of a peptide or an epitope of the human NP1 protein, hereinafter peptide of the invention, based in that constituted by a fragment of the zone comprised between amino acids 24 to 229 of the NP1 protein, preferably of a size of 15 amino acids, and belonging, by way of illustration and without limiting the scope of the invention, to the following group: SEQ ID N018 and SEQ ID N019.

The peptide of the invention can be used for the production of an antibody of the invention which can be used for the production, on the one hand, of a drug for a passive immunisation treatment, and on the other hand, of a drug for an active immunisation treatment (generation of endogenous antibodies) in patients with a neurodegenerative disease, preferably with Alzheimer's disease.

Another aspect of the invention consists of the use of an inhibitor compound or agent of the activity of the human NP1 protein, hereinafter use of a compound of the invention, in the production of a drug or pharmaceutical composition for the treatment of human neurological diseases, preferably neurodegenerative diseases, more preferably Alzheimer's disease.

Another particular aspect of the invention consists of the use of a compound of the invention wherein the inhibitor compound is a nucleic acid or polynucleotide that prevents or diminishes the expression of the gene encoding the human NP1 protein.

Another particular aspect of the invention consists of the use of a compound wherein the inhibitor compound is an antibody specific to the human NP1 protein and functionally active, which prevents or diminishes the neurodegenerative activity of the human NP1 protein.

Another particular aspect of the invention consists of the use of a compound wherein the inhibitor compound is the peptide of the invention, in such a way that the drug thereby obtained can be used in a treatment of active immunisation (generation of endogenous antibodies) in patients with a neurodegenerative disease, preferably with Alzheimer's disease.

Another aspect of the invention consists of a pharmaceutical composition or drug for the treatment of neurological diseases, preferably neurodegenerative diseases, hereinafter pharmaceutical composition of the invention, which comprises a therapeutically effective amount of an inhibitor compound or agent of the human NP1 protein, together with, optionally, one or more adjuvants and/or pharmaceutically acceptable vehicles.

The adjuvants and pharmaceutically acceptable vehicles that can be used in said compositions are the adjuvants and vehicles known by experts in the art and commonly used in the production of therapeutic compositions.

In the sense used in the description, the expression "therapeutically effective amount" refers to the amount of the inhibitor agent or compound of the activity of the NP1 protein, calculated to produce the required effect and, in general, will be determined, among other factors, by the inherent properties of the compounds, including the age, condition of the patient, severity of the alteration or disorder, and the route and frequency of administration.

In a particular embodiment, said therapeutic composition is prepared in solid form or in aqueous suspension, in a pharmaceutically acceptable diluent. The therapeutic composition provided by this invention may be administered via any appropriate route of administration, wherefore said composition shall be formulated in the suitable pharmaceutical form for the selected route of administration. In a particular embodiment, administration of the therapeutic composition provided by the invention is carried out parenterally, orally, intraperitoneally, subcutaneously, etc. A revision of the different pharmaceutical forms of drug administration and the excipients required to obtain them can be found, for example, in the "Treaty of Galenic Pharmacy", C. Fauli i Trillo, 1993, Luzán 5, S.A. Ediciones, Madrid.

Another particular aspect of the invention consists of the pharmaceutical composition of the invention wherein the inhibitor compound is a nucleic acid or a polynucleotide that prevents or diminishes the expression of the gene encoding the human NP1 protein and that comprises, at least, a sequence of nucleotides selected between:
a) an antisense sequence of nucleotides specific to the sequence of the gene or mRNA of the NP1 protein,
b) a ribozyme specific to the mRNA of the NP1 protein,
c) an aptamer specific to the mRNA of the NP1 protein,
d) an interference RNA (iRNA) specific to the mRNA of the NP1 protein, and
e) an interference microRNA (iRNA) specific to the mRNA of the NP1 protein.

Another particular aspect of the invention consists of the pharmaceutical composition of the invention wherein the inhibitor compound is an antibody specific to the NP1 protein. This therapeutic composition can be used in a therapeutic procedure of passive immunisation of patients with a neurodegenerative disease, preferably with Alzheimer's disease.

Another particular aspect of the invention consists of the pharmaceutical composition of the invention wherein the inhibitor compound is a peptide of the invention and which can be used in a therapeutic procedure of active immunisation of patients with a neurodegenerative disease, preferably with Alzheimer's disease.

The therapeutic compositions of the invention can be administered in combination with other drugs used in the treatment of neurodegenerative diseases, with a view to acting in a complementary manner or as a reinforcement (for example, with GSK3 inhibitors that reduce the overexpression of NP1 ⁶).

Another object of the invention consists of the use of the pharmaceutical composition of the invention in a treatment method for a mammal, preferably a human being, affected by a neurological disease, preferably a neurodegenerative disease, more preferably Alzheimer's disease, hereinafter use of the pharmaceutical composition of the invention, consisting in the administration of said therapeutic composition which inhibits the neuropathological process.

### DESCRIPTION OF THE DRAWINGS

**Figure 1** **- The soluble oligomers of Ab 1-42 increase the expression of NP1 in primary cultures of cortical neurons. A)** Representative Western Blot test that shows the progress over time of the effect of a maximum concentration (20 □M) of oligomers of Ab on the levels of proteins NP1 and Actin. **B)** Quantitative analysis showing the concentration-dependent effect of the soluble oligomers of Ab 1-42 (10 and 20 □M) on the levels of NP1 protein. The densitometric values of the bands corresponding to the immunoreactivity of NP1 were normalised with the respective values of the actin band. The proportion between NP1 and actin is expressed as a percentage of the control values. The values represent the mean ± the standard error of the mean of, at least, three independent experiments. * p<0.05, significantly different from the control values (Variance analysis of one route and Bonferroni comparison between groups).
**Figure 2** **- The silencing of NP1 expression prevents the reduction of synapsis produced by Ab.** The expression of NP1 was silenced through RNA interference (RNAi) by means of lentiviral transduction. Primary cultures of cortical neurons were transduced with the control lentiviral vector pLVTHM-shRandom or with the lentiviral vector of RNAi directed against NP1, pLVTHM-shRNAi-NP1 (see Material and Methods, sequences SEQ ID NO 4 and 5). Five microlitres of lentiviral particles were added to the cortical neurons at the moment of seeding. At 5 days of culture, the cells were treated during 48 hours with vehicle (V) or with oligomers of Ab 1-42 (20 □M). **A)** Representative Western Blot test showing that the soluble oligomers Ab 1-42 produce a significant reduction in the levels of synaptophysin and that the silencing of NP1 is capable of reversing this effect. Immunoreactivity against Actin was used as protein load control. **B)** Quantitative analysis of the effects of the soluble oligomers Ab 1-42 and of the silencing of NP1 by RNAi on the levels of synaptophysin. The densitometric values of the bands corresponding to immunoreactivity of synaptophysin were normalised with the respective values of the actin band. The proportion between NP1 and actin is expressed as a percentage of the control values. The values represent the mean ± the standard error of the mean of at least three independent experiments. * p<0.05, significantly different from the control values (t test of independent groups). C, control. V, vehicle.
**Figure 3** **- NP1 levels are increased in the brains of patients with Alzheimer's disease.** Protein analysis in tissue of the hippocampus verifying that the amount of the 54 KDa band corresponding to NP1 is increased in the brains of two (2) patients diagnosed with Alzheimer's disease (AD) compared to the tissue of control patients (C). The specificity of the immunoreactivity of NP1 in human tissue is demonstrated because the band disappears after the pre-absorption of the antibody with recombinant NP1 protein (Pr).
**Figure 4** **- The silencing of NP1 expression by RNAi increases the levels of pre-synaptic (Synaptophysin) and post-synaptic (PSD95) proteins in excitor synapses.** The silencing of NP1 by RNAi, but not the overexpression of NP1, produces a significant increase in the levels of synaptophysin, represented in **A)** and of PSD95, represented in **B)**. The densitometric values of teh bands corresponding to the immunoreactivity of synaptophysin and PSD95 were normalised with the respective values of the actin band. The proportions synaptophysin/actin and PSD95/actin are expressed as a percentage of the control values. The values represent the mean ± the standard error of the mean of at least three independent experiments. * p<0.05, significantly different from the control values (variance analysis of one route with Bonferroni's comparison of groups).
**Figure 5** **- The silencing of NP1 expression by RNAi increases neuronal excitability measured by oscillations in the level of intracellular calcium. A)** represents the spontaneous oscillation of intracellular calcium shown by teh cortical neurons in culture treated with the control lentiviral vector pVLTHMshRandom. **B)** The cortical neurons treated with the lentiviral vector pVTLHM-shNP1 to silence NP1 show a significant increase in the spontaneous oscillation of intracellular calcium concentration which indicates that the silencing of NP1 provokes an increase in neuronal excitability that depends on receptors of excitor amino acids. The treatment with tetradotoxin 5 µM completely blocks the oscillations indicating that they are the result of neurotransmission activity. The results are expressed in % of the basal value of the fluorescence of Fura-2 at 340/380 and are the average of at least ten cortical cells.
**Figure 6** **- A polyclonal antibody directed against epitopes that are found between amino acids 24 to 229 of the protein sequence of the human NP1 protein immunoprecipitates NP1.** The immunoprecipitation test was carried out on SH-SY5Y cells that permanently overexpress NP1. The total lysate was incubated with the antibody against NP1 and the immunoprecipitation was carried out with protein G bound to sepharose particles. The figure is a representative Western Blot showing the immunoreactivity of NP1 in the total lysate (lane 1), in the supernatant after immunoprecipitating (lane 2), and in the immunoprecipitate with an antibody against amino acids 210-224 of the human NP1 protein (lane 3).

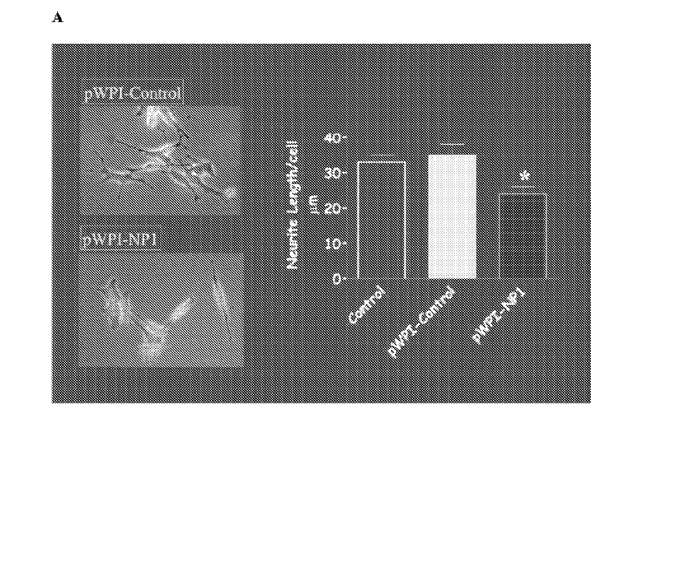

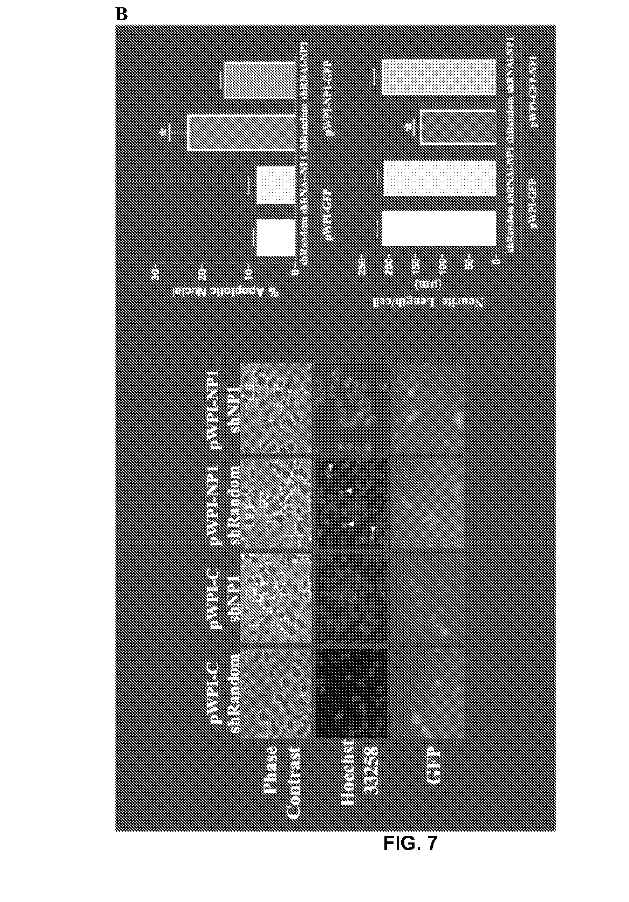

**Figure 7** **- The overexpression of NP1 reduces neurite length and increases apoptosis, whereas the silencing of NP1 blocks these effects. A)** shows the effect of NP1 overexpression in human neuroblastoma cells SHSY5Y. These cells were transduced with the control lentiviral vector pWPI which expresses green fluorescent protein (GFP), or with the lentiviral vector pWPI-NP1 which in addition to GFP, overexpresses NP1. The cells transduced with this last vector, if they express GFP (in green), also overexpress NP1, which in turn produces a significant reduction in the length of its neuritic prolongations. **B)** shows the effect of the overexpression of NP1 and silencing of this overexpression in cortical neurons in culture. The cortical neurons were treated with double infection: first with the control lentivirus (pWPI-C) or with the lentivirus that overexpresses NP1 (pWPI-NP1) and immediately afterwards with the control silencing lentivirus (shRandom) or with the one of RNAi of NP1 (shNP1). The overexpression of NP1 increases the number of apoptotic nuclei measured with Hoechst stain and notably reduces the length of the neurites. The silencing of the gene significantly prevents these effects of NP1.

### EXAMPLES OF THE INVENTION

### Example 1 - The soluble oligomers of the Ab peptide increase the expression of NP1 which induces apoptosis and neuronal neurotoxicity.

In favour of this hypothesis, the results obtained demonstrate that the exposure of primary cultures of cortical neurons to soluble oligomers of the Ab peptide increase the expression of NP1 before inducing apoptotic death (Figure 1). In addition, it has also been proven that NP1 is an essential factor for the production of neurotoxicity by Ab. The experiments establish that Ab causes an increase in the amount of NP1 which in turn provokes loss of synapsis and neuronal damage. The loss of synaptic contacts between neurons is directly related to memory loss. On the contrary, when the increase in NP1 is blocked through gene silencing by interference RNA (see Material and Methods, the data shown in the figures and subsequent examples of the invention were carried out by means of the RNAi of SEQ ID N04 and 5, while similar results although with lower levels of silencing (between 30-70%) were also carried out with two different pairs of RNAi, the sequences SEQ ID N08 and 9 and SEQ ID NO12 and 13, respectively) damage to synapsis is impeded and the appearance of the neurotoxic effects of Ab is prevented (Figure 2). However, in the absence of Ab 1-42, if the neurons are treated with NP1 the neuropathological process induced by Ab is reproduced. It has also been observed that the amount of NP1 is much higher in the brains of patients with Alzheimer's (AD) than in control brains (Figure 3). In brains diagnosed with Alzheimer's the NP1 protein is found in the damaged neuronal dendrites, where it associates with synaptic proteins such as SNAP-25 and synaptophysin. These results establish that NP1 plays a fundamental role in the neurotoxicity provoked by the soluble oligomers of the amyloid beta protein in Alzheimer's disease.

### Example 2.- The gene silencing RNAi of NP1 diminish neuronal apoptosis, induce neuritic growth, increase the number of synapses and increase neuronal excitability.

Using lentiviral vectors of NP1 overexpression and silencing it has been demonstrated that the overexpression of NP1 in primary cultures of cortical neurons activates and increases the number of apoptotic nuclei and reduces neuritic growth. At the same time, cotransduction of NP1 with the silencer of expression shRNAi-NP1 (SEQ ID NO 4 and 5), significantly blocks these effects (Figure 7).

In addition, it has been shown that when the expression of the NP1 protein is reduced through gene silencing by RNAi a significant increase occurs in the synaptic proteins PSD95 and synaptophysin which in previous studies have proven to be reduced in models of Alzheimer's disease, the same as when NP1 is expressed through vectors (Figures 4A and 4B). The increase in PSD95 and synaptophysin indicates that the reduction of NP1 produces an increase in the number of excitor synapses, which constitute evidence of a recovery or maintenance of memory-related parameters.

On a separate note, it has been demonstrated that the gene silencing of NP1 expression, by means of the expression shRNAi-NP1 (SEQ ID NO 4 and 5), produces an increase in neuronal excitability measured by oscillations in intracellular calcium levels measured in primary cultures of cortical neurons (Figure 5). The oscillations in intracellular calcium concentration in neurons where NP1 expression has been silenced by shRNAi are much greater (B) than in control neurons (A) (Figure 5). Also, it is shown that the oscillations in intracellular calcium concentrations are due to synaptic activity because Tetradotoxin, a sodium channel-blocker that eliminates synaptic activity, reduces them markedly (Figure 5B).

**Example 3 - Peptide sequences of NP1 as antigens for use as a vaccine through active immunisation or to generate antibodies for use through passive immunisation to reduce the expression of NP1.** The protein NP1 has 432 amino acids including a secretion or signal sequence of approximately 16-23 amino acids on the amino terminal side. NP1 is expressed fundamentally in the nervous system, whereas other proteins of the pentraxin family are also expressed in other organs. The amino acids found between 229 and 432 have a high homology with other proteins of the family of pentraxins. However, the zone between amino acids 24 and 229 is the most characteristic of NP1 and the one that is most distinct from other proteins such as NP2 and NP3. Also, it is predicted that in this zone there are two supercoiled domains that are confined within amino acids 33-79 and 105-207 and which are those that probably allow NP1 to interact or bind with other proteins. From the zone between amino acids 24 to 229 various sequences have been selected of 15 amino acids in length with antigenic capacity to generate antibodies against this zone of the NP1 protein (see Material and Methods).

Thus, it has been verified that the antibodies directed against epitopes that are found between amino acids 24 to 229 of the sequence of the human NP1 protein (NPTX1_HUMAN, SwissProt primary accession number Q15818) recognise the native and denatured forms of NP1 and are capable of immunoprecipitating NP1, specifically with the antibody generated against the peptide corresponding to amino acids 210-224 (SEQ ID N018) (Figure 6) (Figure 6). The capacity of these antibodies to immunoprecipitate NP1 predict that both the passive immunisation with anti-peptide antibodies whose sequence is within the zone between amino acids 24 to 229, as well as the generation of endogenous antibodies by means of antigenic peptides contained within the same zone, will reduce the amount of NP1. The procedure for obtaining the antibodies against peptides of zone 24 to 229 of the NP1 protein involves conjugating peptides of 15 amino acids with a suitable carrier and injecting them subcutaneously in the presence of an adjuvant (See Material and Methods).

The results of the invention indicate that the overexpression of NP1 reduces neuritic growth in neuroblastoma cells SH-SY5Y (Figure 7A). Likewise, the overexpression of NP1 increases apoptosis and reduces neurite length in cortical neurons in primary cultures (Figure 7 B). NP1 silencing by RNAi blocks these toxic effects of NP1 overexpression (Figure 7B).

### Materials and Methods

Both overexpression of NP1 as well as silencing of the gene has been carried out through transduction with lentiviral vectors in primary cultures of cortical neurons. The bicistronic self-inactivating lentiviral vectors, pWPI and pLVTHM were used, in conjunction with packaging plasmids and second generation capsid ³⁵. To do this, the sequence encoding NP1 was cloned from rat brain cDNA (Quick Clone cDNA, Invitrogen) through PCR and was inserted into the pWP1 vector between the Pmel restriction site through ligation of blunt ends. At the same time, the gene silencing vector of NP1, pLVTHM-shRNAi-NP1-GFP was built to express short interference RNAs (shRNAi). After several tests with various DNA sequences to check the efficiency of gene silencing, it was proven that the shRNAis directed against sequence 1 to 19 bases of the cDNA of NP1 "GAGCTCCAGATCATGAGAA" (SEQ ID N01) made it possible to reduce approximately 70% of the expression of NP1 protein. Other sequences used and checked to also produce silencing of the gene expression of NP1 in a range between 70 and 30% are as follows: sequence 2:"GCGGACCAACTACATGTAT" (SEQ ID N02); sequence 3:"GAGATACTCATTAACGACA" (SEQ ID N03).

In general, two complementary DNA oligonucleotides are hybridised to produce a double-chain DNA fragment that encodes an RNA chain of 19 nucleotides in the sense orientation with a loop of 9 nucleotides and a chain of 19 anti-sense nucleotides directed against the sequence of NP1. Thus, the sequence 1 sense of shRNAi against NP1 (SEQ ID N01) is as follows: 5'-gatcccc GTACAGCCGCCTCAATTCT ttcaagaga AGAATTGAGGCGGCTGTAC ttttt-'3 (sense) (SEQ ID N04), and sequence 2 anti-sense is 5'-agctaaaaa GTACAGCCGCCTCAATTCT tctcttgaa AGAATTGAGGCGGCTGTAC ggg-3' (SEQ ID N05). The sequence in capitals is the target sequence of NP1 which corresponds to the bases 1004 to 1022 of the mRNA of rat NP1 (SEQ ID NO1) (Genbank Accession number U 18772).

On a separate note, the corresponding sequence of shRNAi directed against the homologous human form of sequence SEQ ID N01 of NP1 is: 5'-gatcccc GCAGTACAGCCGCCTCAAT ttcaagaga ATTGAGGCGGCTGTACTGC ttttt-'3 (sense) (SEQ ID N06), and 5'- 33 agctaaaaa GCAGTACAGCCGCCTCAAT tctcttgaa ATTGAGGCGGCTGTACTGC ggg-3' (antisense) (SEQ ID N07), which corresponds to the bases 602 to 620 of the mRNA of human NP1 (Genbank Acession number NM_002522).

The other sequences verified to also produce silencing of the gene expression of NP1 within a range of 70 and 30% are as follows:
- sequences against sequence "GCGGACCAACTACATGTAT" (SEQ ID N02), where sequence 1 sense of shRNAi is 5'-gatcccc GCGGACCAACTACATGTAT ttcaagaga ATACATGTAGTTGGTCCGC ttttt-'3 (SEQ ID N08), and sequence 2 antisense is as follows 5'-agctaaaaa GCGGACCAACTACATGTAT tctcttgaa ATACATGTAGTTGGTCCGC ggg- 3' (SEQ ID N09). The sequence in capitals is the target sequence of NP1 which corresponds to the bases 1259 to 1277 of the mRNA of rat NP1 (Genbank Accession number U18772). The corresponding sequence of shRNAi against the human form of NP1 is 5'-gatcccc GCGGACCAACTATATGTAT ttcaagaga ATACATATAGTTGGTCCGC ttttt-'3 (sense) (SEQ ID NO10), and 5'-agctaaaaa GCGGACCAACTATATGTAT tctcttgaa ATACATATAGTTGGTCCGC ggg-3' (antisense) (SEQ ID NO11) which corresponds to the bases 860 to 878 of the mRNA of human NP1 (Genbank Accession number NM_002522).
- sequences against sequence "GAGATACTCATTAACGACA" (SEQ ID N03), where sequence 1 sense of the shRNAi is 5'-gatcccc GAGATACTCATTAACGACA ttcaagaga TGTCGTTAATGAGTATCTC ttttt-'3 (SEQ ID N012), and sequence 2 antisense is the following 5'- agctaaaaa GAGATACTCATTAACGACA tctcttgaa TGTCGTTAATGAGTATCTC ggg-3' (SEQ ID N013). The sequence in capitals is the target sequence of NP1 which corresponds to the bases 1434 to 1452 of the mRNA of rat NP1 (Genbank Accession number U18772). The corresponding sequence of shRNAi against the human form of NP1 is 5'-gatcccc GAGATCCTCATCAATGACA ttcaagaga TGTCATTGATGAGGATCTC ttttt-'3 (sense) (SEQ ID NO14), and 5'- agctaaaaa GAGATCCTCATCAATGACA tctcttgaa TGTCATTGATGAGGATCTC ggg-3' (antisense) (SEQ ID NO15) which corresponds to the bases 1035 to 1053 of the mRNA of human NP1 (Genbank Acession number NM_002522).

The shRNAi that was designed for use as control is a random sequence (Random) that was introduced into the control lentiviral vector. The sequence of the Random-shRNAi is: 5'- gatcccc GCAGTGCAATATCGGAAAC ttcaagaga GTTTCCGATATTGCACTGC ttttt-3' (sense) (SEQ ID NO16) and 5'-agctaaaaa GCAGTGCAATATCGGAAAC tctcttgaa GTTTCCGATATTGCACTGC ggg -3' (antisense) (SEQ ID N017).

The DNA duplex of shRNAi-NP1 and shRNAi-Random were cloned between the restriction sites Hindlll and Bglll of the vector pSUPER.retro. After confirming that shRNAi is capable of silencing the expression of NP1, the previous sequences were sub-cloned in the lentiviral vector pLVTHM between restriction sites EcoR1-Cla1.

### Production and titration of the lentiviral vectors

The viral particles are pseudotyped with glycoprotein G of the vesicular stomatitis virus and are obtained by means of transitory transfection in 293T cells following the standard procedure described by the laboratory of Dr. Trono ³⁶. The viral particles are concentrated by centrifuging. The titration to determine the concentration of viral particles is carried out by means of transduction of 293T cells with seriated dilutions of the viral concentrate and subsequent counting by flow cytometry of the number of cells that express the marker encoded by the virus which is green fluorescent protein (GFP). The values of viral concentration obtained through this procedure are in the range of de 1-2x 10⁹ transduction units per millilitre (TU/ml).

### Transduction of primary cultures of cortical neurons with the lentiviral vectors.

The lentiviral particles are added to the cultures of cortical neurons immediately after seeding in plates in some experiments, or after maturation in other experiments. The amount of particles per titration used in our experiments is in the range of 2-10x10⁶ TUs in a volume of between 2 and 5 µl of the viral concentrate. The percentage of cortical neurons that express GFP 48 hours after transduction is 80-90%.

### Primary cultures of cortical neurons.

The cultures are prepared using E18 rat foetuses of the Sprague-Dawley strain. The culture procedure basically follows the protocol described by Enguita y cols ¹⁶. The procedure consists of: dissecting the cortex, chemical dissociation of the cells in the presence of trypsin and DNAse I, subsequent dilution in the Eagle Basal culture medium supplemented with 2 mM glutamine, 25 mM potassium and 10% bovine foetal serum and the seeding of the cells in wells coated in poly-L-lysine (10 □g/ml) at a density of 9x10⁵ cells/cm² in medium supplemented with glucose (25 mM). To prevent glial proliferation, 10 µM arabinoside cytosine is added 72 hours after seeding and the experiments are carried out in cultures during 8 days *in vitro.*

### Preparation of the soluble oligomers of Ab 1-42.

The soluble oligomers of Ab 1-42, also known as amyloid derived diffusible ligands (ADDLs) are prepared following the standard procedures described by various groups ³⁷⁻³⁹.

### Immunoreactivity analysis of NP1 proteins and synaptophysin by SDS-PAGE and Western blot.

Obtaining the proteins from primary cultures and the cultures of cell lines is achieved after the various experimental treatments by following standard procedures. The cells are solubilised in SDS buffer (62.5 mM Tris-HCl pH6.8, 2% SDS, 10% glycerol, 2.5 mM EDTA, 75 mM DTT and 0.001 % bromophenol blue). Next, the proteins are separated by means of electrophoresis in denaturing gels of SDS-polyacrylamide following standard procedures. The separated polypeptides are passed onto PVDF membranes (Millipore) activated by electro-transfer. The non specific bond is blocked by incubating the membranes in a TBS Tween solution with 5% powdered skimmed milk. In order to specifically detect NP1, the membranes are incubated in the presence of monoclonal or polyclonal antibodies against rat NP1 (1:1500, Transduction Laboratories) in a solution of 3% BSA in TBST. In order to detect synaptophysin, the membranes are incubated with the monoclonal antibody SY38 (1:1000, Chemicon). Next, the membranes are incubated with secondary antibodies conjugated with peroxidase. As protein load control immunodetection of actin is used by means of a polyclonal rabbit antibody against actin 20-33 (1:3000, Sigma). Viewing of the immunoreactive proteins was carried out using an improved chemiluminescence system (Supersignal WestDura, Pierce), and the detection of bands of immunoreactivity was carried out using the image analysis system VersaDoc Model 5000 (Bio-Rad). The intensity of the bands obtained is quantified by densitometry using the computer analysis programme Quantity One (Bio-Rad). The densitometric values of the immunoreactivity bands of NP1 and synaptophysin are normalised with the value of the corresponding actin band.

### Determination of the concentration of intracellular calcium in cortical neurons in culture.

The cortical neurons are loaded with a concentration of 5 µM of the calcium indicator Fura-2-AM (Molecular Probes) during one hour at room temperature in a saline solution buffered with 10 mM HEPES (pH 7.4). The measurement of fluorescence induced by calcium entry is carried out in an inverted epifluorescence microscope with a 20X fluorite objective. The emission fluorescence is determined at 510 nm in each neuron following excitation with an alternating light beam of 340 nm and 380 nm using an emission filter of 390 nm. The fluorescence generated by the binding of Fura-2 with intracellular calcium is expressed as F340/F380 (proportion of emission fluorescence at 340/emission fluorescence at 380).

### Production of antibodies directed against epitopes found between amino acids 24 to 229 of the sequence of human NP1 protein.

By analysing the hydrophobicity pattern of the fragment that incorporates amino acids 24 to 229 of the sequence of human NP1 protein, the peptides of 15 amino acids with antigenic capacity were selected:
- Peptide NP1_210-224: QRISELEKGQKDNRP (amino-acids 210-224 rat sequence, SEQ ID N018),
- Peptide NP1_100-115: GEARSGGGRKQPGSG (amino-acids 100-115 sequence rat, SEQ ID N019)

After synthesis and purification, said synthetic peptides were attached to carrier proteins such as KLH or BSA following standard procedures. Next, they were injected together with an adjuvant in order to immunise and to produce polyclonal antibodies in rabbits. The level of anti-peptide antibodies of NP1 was determined in serum samples by indirect ELISA on plates where previously each one of the synthetic peptides had been fixed. Finally, the antibodies were purified in an immunoaffinity column following standard procedures.

For further details concerning the materials and methods refer to the study of the inventors (Maria A. Abad, Marta Enguita, Nuria DeGregorio-Rocasolano, Isidre Ferrer, and Ramón Trullas. Neuronal Pentraxin 1 Contributes to the Neuronal Damage Evoked by Amyloid-□ and Is Overexpressed in Dystrophic Neurites in Alzheimer's Brain. The Journal of Neuroscience, December 6, 2006, 26(49):12735-12747).

### Bibliography

1.- M. Vila and S. Przedborski, Nat. Rev. Neurosci. 4, 365-375 (2003).
2.- R. R. Buss, W. Sun, R. W. Oppenheim, Annu. Rev. Neurosci. 29:1-35.,10 1-35 (2006).
3.- G. Kroemer and J. C. Reed, Nat. Med. 6, 513-519 (2000).
4.- D. E. Bredesen, R. V. Rao, P. Mehlen, Nature. 443, 796-802 (2006).
5.- P. Liang and A. B. Pardee, Science 1992, 967-971 (1992).
6.- N. DeGregorio-Rocasolano, T. Gasull, R. Trullas, J. Biol. Chem. 276, 15 796-803 (2001).
7.- A. K. Schlimgen, J. A. Helms, H. Vogel, M. S. Perin, Neuron 14, 519-526 (1995).
8.- A. R. Goodman et al., Cytokine. Growth Factor. Rev. 7, 191-202 (1996).
9.- D. C. Dodds, I. A. Omeis, S. J. Cushman, J. A. Helms, M. S. Perin, Journal of Biological Chemistry 272, 21488-21494 (1997).
10.- Y. C. Hsu and M. S. Perin, Genomics 28, 220-227 (1995).
11.- C. C. Tsui et al., J. Neurosci. 16, 2463-2478 (1996).
12.- R. J. O'Brien et al., Neuron 23, 309-323 (1999).
13.- D. Xu et al., Neuron 39, 513-528 (2003).
14.- R. O'Brien et al., J Neurosci. 22, 4487-4498 (2002).
15.- B. Chen and J. L. Bixby, J Comp Neurol. 481, 391402 (2005).
16.- M. Enguita, N. DeGregorio-Rocasolano, A. Abad, R. Trullas, Mol. Pharmacol. 67, 1237-1246 (2005).
17.- A. Takashima, K. Noguchi, K. Sato, T. Hoshino, K. Imahori. Proc. Natl. Acad. Sci. U.S.A 90, 7789-7793 (1993).
18.- R. V. Bhat, S. L. Budd Haeberlein, J. Avila, J Neurochem. 89, 1313-1317 (2004).
19.- C. L. Sayas, J. Avila, F. Wandosell, Biochim. Biophys. Acta 1582, 144-153 (2002).
20.- S. Sanchez et al., J Neurochem. 78, 468-481 (2001).
21.- J. R. Munoz-Montano, F. Lim, F. J. Moreno, J. Avila, J. Diaz-Nido, J Alzheimers. Dis. 1, 361-378 (1999).
22.- F. Q. Zhou, J. Zhou, S. Dedhar, Y. H. Wu, W. D. Snider, Neuron 42, 897-912 (2004).
23.- E. D. Roberson and L. Mucke, Science. 314, 781-784 (2006).
24.- D. M. Walsh and D. J. Selkoe, J. Neurochem. 101, 1172-1184 (2007).
25.- C. Haass and D. J. Selkoe, Nat. Rev. Mol. Cell Biol. 8,101-112 (2007).
26.- D. M. Walsh and D. J. Selkoe, Neuron 44, 181-193 (2004).
27.- J. Hardy and D. J. Selkoe, Science 297, 353-356 (2002).
28.- C. W. Cotman and J. H. Su, Brain Pathol. 6, 493506 (1996).
29.- Y. Morishima et al., J. Neurosci. 21, 7551-7560 (2001).
30.- D. T. Loo et al., Proc. Natl. Acad. Sci. U.S.A 90, 7951-7955 (1993).
31.- K. J. Ivins, E. T. Bui, C. W. Cotman, Neurobiol. Dis. 5, 365-378 (1998).
32.- M. A. Abad, M. Enguita, N. DeGregorio-Rocasolano, I. Ferrer, R. Trullas, J. Neurosci. 26, 12735-12747 (2006).
33.- F. Kamenetz et al., Neuron 37, 925-937 (2003).
34.- D. M. Walsh et al., Nature 416, 535-539 (2002).
35.- M. Wiznerowicz and D. Trono, J. Virol. 77, 89578961 (2003).
36.- R. Zufferey et al., J.Virol. 72, 9873-9880 (1998).
37.- M. P. Lambert et al., Proc. Natl. Acad. Sci. U.S.A 95, 6448-6453 (1998).
38.- K. N. Dahlgren et al., J Biol.Chem. 277, 3204632053 (2002).
39.- W. L. Klein, Neurochem. Int. 41, 345-352 (2002).

## Claims

1. Compound which can be used for the production of drugs or pharmaceutical compositions for the treatment or prevention of human neurological diseases, preferably neurodegenerative diseases, **characterised in that** it consists of an inhibitor compound or agent of the activity of human NP1 protein.

2. Compound according to claim 1 **characterised in that** the human NP1 protein presents the sequence NPTX1_HUMAN, SwissProt primary accession number Q15818 or a functionally equivalent variant thereof.

3. Compound according to claim 1 **characterised in that** the inhibitor compound is a nucleic acid or polynucleotide that prevents or diminishes the expression of the gene encoding human NP1 protein and that includes, at least, a sequence of nucleotides selected between:
a) an antisense sequence of nucleotides specific to the sequence of the gene or mRNA of the NP1 protein,
b) a ribozyme specific to the mRNA of the NP1 protein,
c) an aptamer specific to the mRNA of the NP1 protein,
d) an interference RNA (shRNAi) specific to the mRNA of the NP1 protein, and
e) a microRNA specific to the mRNA of the NP1 protein.

4. Compound according to claim 3 **characterised in that** the shRNAi of d) is an shRNAi that preferably binds to the fragment sequence of RNAm of NP1 GAGCTCCAGATCATGAGAA (SEQ ID N01) or to another fragment that comprises this sequence.

5. Compound according to claim 4 **characterised in that** the shRNAi is an shRNAi expressed by the following pair of nucleotides: 5'-gatcccc GTACAGCCGCCTCAATTCT ttcaagaga AGAATTGAGGCGGCTGTAC ttttt-'3 (SEQ ID N04, sense) and 5'-agctaaaaa GTACAGCCGCCTCAATTCT tctcttgaa AGAATTGAGGCGGCTGTAC ggg-3' (SEQ ID N05, antisense).

6. Compound according to claim 3 **characterised in that** the shRNAi of d) is an shRNAi that preferably binds to the fragment sequence of RNAm of NP1 GCGGACCAACTACATGTAT (SEQ ID N02) or to another fragment that comprises this sequence.

7. Compound according to claim 6 **characterised in that** the shRNAi is an shRNAi expressed by the following pair of nucleotides: 5'-gatcccc GCGGACCAACTACATGTAT ttcaagaga ATACATGTAGTTGGTCCGC ttttt-'3 (SEQ ID NO8, sense) and 5'-agctaaaaa GCGGACCAACTACATGTAT tctcttgaa ATACATGTAGTTGGTCCGC ggg-3' (SEQ ID N09, antisense).

8. Compound according to claim 6 **characterised in that** the shRNAi is an shRNAi expressed by the following pair of nucleotides: 5'-gatcccc GCGGACCAACTATATGTAT ttcaagaga ATACATATAGTTGGTCCGC ttttt-'3 (SEQ ID NO10, sense), and 5'-agctaaaaa GCGGACCAACTATATGTAT tctcttgaa ATACATATAGTTGGTCCGC ggg-3' (SEQ ID N011, antisense).

9. Compound according to claim 3 **characterised in that** the shRNAi of d) is an shRNAi that preferably binds to the fragment sequence of RNAm of NP1 GAGATACTCATTAACGACA (SEQ ID N03) or to another fragment that comprises this sequence.

10. Compound according to claim 8 **characterised in that** the shRNAi is an shRNAi expressed by the following pair of nucleotides: 5'-gatcccc GAGATACTCATTAACGACA ttcaagaga TGTCGTTAATGAGTATCTC ttttt-3' (SEQ ID NO12, sense) and 5'-agctaaaaa GAGATACTCATTAACGACA tctcttgaa TGTCGTTAATGAGTATCTC ggg-3' (SEQ ID N013, antisense).

11. Compound according to claim 8 **characterised in that** the shRNAi is an shRNAi expressed by the following pair of nucleotides: 5'-gatcccc GAGATCCTCATCAATGACA ttcaagaga TGTCATTGATGAGGATCTC ttttt-3' (SEQ ID NO14, sense), and 5'-agctaaaaa GAGATCCTCATCAATGACA tctcttgaa TGTCATTGATGAGGATCTC ggg-3' (SEQ ID N015, antisense).

12. Compound according to claim 1 **characterised in that** the inhibitor compound is a vector that comprises a nucleic acid or polynucleotide of the invention which prevents or diminishes the expression of the gene encoding human NP1 protein, whether an expression vector or a transfer vector.

13. Compound according to claim 12 **characterised in that** the expression vector is a viral expression vector, preferably a lentivirus capable of expressing inside a cell a nucleotide that inhibits the expression of human NP1 protein.

14. Compound according to claim 13 **characterised in that** the lentivirus is the vector pLVTHM-shRNAi-NP1 which comprises, at least, one of the following pairs of sequences:
- sequences SEQ ID NO 4 and 5,
- sequences SEQ ID NO 6 and 7,
- sequences SEQ ID NO 8 and 9,
- sequences SEQ ID NO 10 and 11,
- sequences SEQ ID NO 12 and 13, and
- sequences SEQ ID NO 14 y 15.

15. Compound according to claim 1 **characterised in that** the inhibitor compound is an antibody specific to human NP1 protein and functionally active which prevents or diminishes the neurodegenerative activity of human NP1 protein, whether monoclonal or polyclonal.

16. Compound according to claim 15 **characterised in that** the antibody is specific to an epitope found between amino acids 24 to 229 of the sequence of human NP1 protein (NPTX1_HUMAN, SwissProt primary accession number Q15818), preferably an epitope or peptide of 15 amino acids in size.

17. Compound according to claim 16 **characterised in that** the antibody is specific to an epitope with a sequence of amino acids belonging to the following group: SEQ ID N018 and SEQ ID N019.

18. Peptide or epitope which can be used in the production of drugs or pharmaceutical compositions for the treatment of human neurological diseases **characterised in that** it consist of a fragment of amino acids of the zone comprised between amino acids 24 to 229 of the human NP1 protein (NPTX1_HUMAN, SwissProt primary accession number Q15818), preferably of a size of 15 amino acids.

19. Peptide according to claim 18 **characterised in that** the sequence of amino acids of the peptide belongs to the following group: SEQ ID N018 and SEQ ID NO19.

20. Use of a compound according to claims 1 to 17 or of a peptide according to claims 18 and 19 in the production of a drug or pharmaceutical composition for the treatment of human neurological diseases, preferably neurodegenerative diseases, more preferably Alzheimer's disease.

21. Pharmaceutical composition or drug for the treatment of neurological diseases, preferably neurodegenerative diseases **characterised in that** it comprises a therapeutically effective amount of an inhibitor compound or agent of the human NP1 protein, together with, optionally, one or more adjuvants and/or pharmaceutically acceptable vehicles.

22. Pharmaceutical composition according to claim 21 **characterised in that** the inhibitor compound is a nucleic acid or polynucleotide that prevents or diminishes the expression of the gene encoding the human NP1 protein and that comprises, at least, a sequence of nucleotides selected between:
a) an antisense sequence of nucleotides specific to the gene or mRNA of the NP1 protein,
b) a ribozyme specific to the mRNA of the NP1 protein,
c) an aptamer specific to the mRNA of the NP1 protein,
d) an interference RNA (iRNA) specific to the mRNA of the NP1 protein, and
e) an interference microRNA (iRNA) specific to the mRNA of the NP1 protein.

23. Pharmaceutical composition according to claim 21 **characterised in that** the inhibitor compound is an antibody specific to the NP1 protein.

24. Pharmaceutical composition according to claim 21 **characterised in that** the inhibitor compound is a peptide according to claims 18 and 19.

25. Use of the pharmaceutical composition according to claims 21 to 24 in a treatment method for a mammal, preferably a human being, affected by a neurological disease, preferably a neurodegenerative disease, more preferably Alzheimer's disease, consisting of administering said therapeutic composition which inhibits the neuropathological process.

26. Use of the pharmaceutical composition according to claim 25 **characterised in that** the treatment method consists of a therapeutic procedure of passive immunisation.

27. Use of the pharmaceutical composition according to claim 25 **characterised in that** the treatment method consists of a therapeutic procedure of active immunisation.


SEQUENCE LISTING

<110>  CONSEJO SUPERIOR DE INVESTIGACIONES CIENTÍFICAS

<120>  NP1 ACTIVITY REGULATING ELEMENTS USEFUL FOR THE PRODUCTION OF DRUGS FOR THE
TREATMENT OR PREVENTION OF HUMAN NEURODEGENERATIVE DISEASES, RESULTING DRUGS AND USE
THEREOF

<130>  NP1

<160>  19

<170>  PatentIn version 3.3

<210>  1
<211>  19
<212>  DNA
<213>  Artificial sequence

<220>
<223>  1004-1022 nt domain of NP1 from rat

<400>  1
gtacagccgc ctcaattct
                    19

<210>  2
<211>  19
<212>  DNA
<213>  Artificial sequence

<220>
<223>  1259-1277 nt domain of NP1 from rat

<400>  2
gcggaccaac tacatgtat                                                    19

<210>  3
<211>  19
<212>  DNA
<213>  Artificial sequence

<220>
<223>  1434-1452 nt domain of NP1 from rat

<400>  3
gagatactca ttaacgaca                                                    19

<210>  4
<211>  59
<212>  DNA
<213>  Artificial sequence

<220>
<223>  shRNAi 1r sense of NP1 1004/1022

<400>  4
gatccccgta cagccgcctc aattctttca agagaagaat tgaggcggct gtactttttt      59

<210>  5
<211>  59
<212>  DNA
<213>  Artificial sequence

<220>

<223>  shRNAi 1r antisense of NP1 1004-1022

<400>  5
agctaaaaag tacagccgcc tcaattcttc tcttgaaaga attgaggcgg ctgtacggg      59


<210>  6
<211>  59
<212>  DNA
<213>  Artificial sequence

<220>
<223>  shRNAi 1h sense of NP1 602-620 nt

<400>  6
gatccccgca gtacagccgc ctcaatttca agagaattga ggcggctgta ctgcttttt      59


<210>  7
<211>  59
<212>  DNA
<213>  Artificial sequence

<220>
<223>  shRNAi 1h antisense of NP1 602-620 nt

<400>  7
agctaaaaag cagtacagcc gcctcaattc tcttgaaatt gaggcggctg tactgcggg      59


<210>  8
<211>  59
<212>  DNA
<213>  Artificial sequence

<220>
<223>  shRNAi 2r sense of NP1 1259-1277 nt

<400>  8
gatccccgcg gaccaactac atgtatttca agagaataca tgtagttggt ccgcttttt      59


<210>  9
<211>  59
<212>  DNA
<213>  Artificial sequence

<220>
<223>  shRNAi 2r antisense of NP1 1259-1277 nt

<400>  9
agctaaaaag cggaccaact acatgtattc tcttgaaata catgtagttg gtccgcggg      59


<210>  10
<211>  59
<212>  DNA
<213>  Artificial sequence

<220>
<223>  shRNAi 2h sense of NP1 860-878 nt

<400>  10
gatccccgcg gaccaactat atgtatttca agagaataca tatagttggt ccgcttttt      59


<210>  11
<211>  59
<212>  DNA

<213>  Artificial sequence

<220>
<223>  shRNAi 2h antisense of NP1 860-878 nt

<400>  11
agctaaaaag cggaccaact atatgtattc tcttgaaata catatagttg gtccgcggg      59


<210>  12
<211>  59
<212>  DNA
<213>  Artificial sequence

<220>
<223>  shRNAi 3r sense of NP1 1434-1452 nt

<400>  12
gatccccgag atactcatta acgacattca agagatgtcg ttaatgagta tctcttttt      59


<210>  13
<211>  59
<212>  DNA
<213>  Artificial sequence

<220>
<223>  shRNAi 3r antisense of NP1 1434-1452 nt

<400>  13
agctaaaaag agatactcat taacgacatc tcttgaatgt cgttaatgag tatctcggg      59


<210>  14
<211>  59
<212>  DNA
<213>  Artificial sequence

<220>
<223>  shRNAi 3h sense of NP1 1035-1053 nt

<400>  14
gatccccgag atcctcatca atgacattca agagatgtca ttgatgagga tctcttttt      59


<210>  15
<211>  59
<212>  DNA
<213>  Artificial sequence

<220>
<223>  shRNAi 3h antisense of NP1 1035-1053 nt

<400>  15
agctaaaaag agatcctcat caatgacatc tcttgaatgt cattgatgag gatctcggg      59


<210>  16
<211>  59
<212>  DNA
<213>  Artificial sequence

<220>
<223>  shRNAi-Random sense

<400>  16
gatccccgca gtgcaatatc ggaaacttca agagagtttc cgatattgca ctgcttttt      59

<210>  17
<211>  59
<212>  DNA
<213>  Artificial sequence

<220>
<223>  shRNAi-Ramdom antisense

<400>  17
agctaaaaag cagtgcaata tcggaaactc tcttgaagtt tccgatattg cactgcggg      59


<210>  18
<211>  15
<212>  PRT
<213>  Artificial

<220>
<223>  NP1_210-224 peptide

<400>  18

Gln Arg Ile Ser Glu Leu Glu Lys Gly Gln Lys Asp Asn Arg Pro
1               5                   10                  15


<210>  19
<211>  15
<212>  PRT
<213>  Artificial sequence

<220>
<223>  NP1_100-115 peptide

<400>  19

Gly Glu Ala Arg Ser Gly Gly Gly Arg Lys Gln Pro Gly Ser Gly
1               5                   10                  15

SEQUENCE LISTING

<110>  CONSEJO SUPERIOR DE INVESTIGACIONES CIENTÍFICAS

<120>  ELEMENTOS REGULADORES DE LA ACTIVIDAD DE NP1 ÚTILES PARA LA
       ELABORACIÓN DE MEDICAMENTOS PARA EL TRATAMIENTO O PREVENCIÓN DE
       ENFERMEDADES HUMANAS NEURODEGENERATIVAS, MEDICAMENTOS ASÍ
       OBTENIDOS Y SUS APLICACIONES

<130>  NP1

<160>  19

<170>  PatentIn version 3.3

<210>  1
<211>  19
<212>  DNA
<213>  Artificial

<220>
<223>  Dominio 1004-1022 nt de NP1 de rata

<400>  1
gagctccaga tcatgagaa                                                    19


<210>  2
<211>  19
<212>  DNA
<213>  Artificial

<220>
<223>  Dominio 1259-1277 nt de NP1 de rata

<400>  2
gcggaccaac tacatgtat                                                    19


<210>  3
<211>  19
<212>  DNA
<213>  Artificial

<220>
<223>  Dominio 1434-1452 nt de NP1 de rata

<400>  3
gagatactca ttaacgaca                                                    19


<210>  4
<211>  59
<212>  DNA
<213>  Artificial

<220>
<223>  shRNAi 1r sentido NP1 1004/1022

<400>  4
gatccccgta cagccgcctc aattctttca agagaagaat tgaggcggct gtactttttt      59


<210>  5
<211>  59
<212>  DNA
<213>  Artificial

<220>

<223>  shRNAi 1r antisentido NP1 1004-1022

<400>  5
agctaaaaag tacagccgcc tcaattcttc tcttgaaaga attgaggcgg ctgtacggg      59


<210>  6
<211>  59
<212>  DNA
<213>  Artificial

<220>
<223>  shRNAi 1h sentido NP1 602-620 nt

<400>  6
   gatccccgca gtacagccgc ctcaatttca agagaattga ggcggctgta ctgcttttt      59


<210>  7
<211>  59
<212>  DNA
<213>  Artificial

<220>
<223>  shRNAi 1h antissentido NP1 602-620 nt

<400>  7
agctaaaaag cagtacagcc gcctcaattc tcttgaaatt gaggcggctg tactgcggg      59


<210>  8
<211>  59
<212>  DNA
<213>  Artificial

<220>
<223>  shRNAi 2r sentido NP1 1259-1277 nt

<400>  8
   gatccccgcg gaccaactac atgtatttca agagaataca tgtagttggt ccgcttttt      59


<210>  9
<211>  59
<212>  DNA
<213>  Artificial

<220>
<223>  shRNAi 2r antisentido NP1 1259-1277 nt

<400>  9
agctaaaaag cggaccaact acatgtattc tcttgaaata catgtagttg gtccgcggg      59


<210>  10
<211>  59
<212>  DNA
<213>  Artificial

<220>
<223>  shRNAi 2h sentido NP1 860-878 nt

<400>  10
   gatccccgcg gaccaactat atgtatttca agagaataca tatagttggt ccgcttttt      59


<210>  11
<211>  59
<212>  DNA

<213>  Artificial

<220>
<223>  shRNAi 2h antisentido NP1 860-878 nt

<400>  11
agctaaaaag cggaccaact atatgtattc tcttgaaata catatagttg gtccgcggg      59


<210>  12
<211>  59
<212>  DNA
<213>  Artificial

<220>
<223>  shRNAi 3r sentido NP1 1434-1452 nt

<400>  12
gatccccgag atactcatta acgacattca agagatgtcg ttaatgagta tctcttttt      59


<210>  13
<211>  59
<212>  DNA
<213>  Artificial

<220>
<223>  shRNAi 3r antisentido NP1 1434-1452 nt

<400>  13
agctaaaaag agatactcat taacgacatc tcttgaatgt cgttaatgag tatctcggg      59


<210>  14
<211>  59
<212>  DNA
<213>  Artificial

<220>
<223>  shRNAi 3h sentido NP1 1035-1053 nt

<400>  14
gatccccgag atcctcatca atgacattca agagatgtca ttgatgagga tctcttttt      59


<210>  15
<211>  59
<212>  DNA
<213>  Artificial

<220>
<223>  shRNAi 3h antisentido NP1 1035-1053 nt

<400>  15
agctaaaaag agatcctcat caatgacatc tcttgaatgt cattgatgag gatctcggg      59


<210>  16
<211>  59
<212>  DNA
<213>  Artificial

<220>
<223>  shRNAi-Random sentido

<400>  16
gatccccgca gtgcaatatc ggaaacttca agagagtttc cgatattgca ctgcttttt      59

<210>  17
<211>  59
<212>  DNA
<213>  Artificial

<220>
<223>  shRNAi-Ramdom antisentido

<400>  17
agctaaaaag cagtgcaata tcggaaactc tcttgaagtt tccgatattg cactgcggg      59


<210>  18
<211>  15
<212>  PRT
<213>  Artificial

<220>
<223>  Péptido NP1_210-224

<400>  18

Gln Arg Ile Ser Glu Leu Glu Lys Gly Gln Lys Asp Asn Arg Pro
1               5                   10                  15


<210>  19
<211>  15
<212>  PRT
<213>  Artificial

<220>
<223>  Péptido NP1_100-115

<400>  19

Gly Glu Ala Arg Ser Gly Gly Gly Arg Lys Gln Pro Gly Ser Gly
1               5                   10                  15